(19) Europäisches Patentamt
European Patent Office
Office européen des brevets

(11) **EP 4 386 622 A1**

(12) **EUROPEAN PATENT APPLICATION**

(43) Date of publication:
**19.06.2024 Bulletin 2024/25**

(21) Application number: **23197705.9**

(22) Date of filing: **15.09.2023**

(51) International Patent Classification (IPC):
**G06N 3/044** (2023.01)       **G06N 3/047** (2023.01)
**G06N 3/049** (2023.01)       **G06N 3/088** (2023.01)

(52) Cooperative Patent Classification (CPC):
**G06N 3/049; G06N 3/044; G06N 3/047;**
**G06N 3/088;** G06N 3/045; G06N 7/01

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **25.11.2022 IN 202221068002**

(71) Applicant: **Tata Consultancy Services Limited**
**Maharashtra (IN)**

(72) Inventors:
• **BANERJEE, Dighanchal**
**700160 Kolkata, West Bengal (IN)**

• **DEY, Sounak**
**700160 Kolkata, West Bengal (IN)**
• **PAL, Arpan**
**700160 Kolkata, West Bengal (IN)**

(74) Representative: **Goddar, Heinz J. et al**
**Boehmert & Boehmert**
**Anwaltspartnerschaft mbB**
**Pettenkoferstrasse 22**
**80336 München (DE)**

Remarks:
Claims 16, 17 are deemed to be abandoned due to non-payment of the claims fees (Rule 45(3) EPC).

(54) **METHOD AND SYSTEM OF SPIKING NEURAL NETWORK-BASED ECG CLASSIFIER FOR WEARABLE EDGE DEVICES**

(57)     This disclosure relates generally to method and system for spiking neural network based ECG classifier for wearable edge devices. Employing deep neural networks to extract the features from ECG signal have high computational intensity and large power consumption. The spiking neural network of the present disclosure obtains a training dataset comprising a plurality of ECG time-series data. The spiking neural network comprise a reservoir-based spiking neural network and a feed forward based spiking neural network. Each of the spiking neural network having a logistic regression-based ECG classifier are trained to classify one or more class labels. The peak-based spike encoder of each spiking neural network obtains a plurality of encoded spike trains from the plurality of ECG time-series. The peak-based spike encoder provides high performance for classifying one or more labels. Efficacy of the peak-based spike encoder for classification is experimentally evaluated with different datasets.

400

obtain by a spiking neural network a training dataset comprising a plurality of ECG time-series data from a plurality of sensors, wherein the spiking neural network comprise at least one of a first neural network and a second neural network — 402

train a logistic regression-based ECG classifier associated with at least one of the first neural network and the second neural network with the plurality of ECG time-series data — 404

processing a plurality of test dataset received as input into at least one of the spiking neural network to obtain associated class label from the logistic regression-based ECG classifier — 406

**FIG. 4**

EP 4 386 622 A1

**Description**

CROSS-REFERENCE TO RELATED APPLICATIONS AND PRIORITY

The present application claims priority to Indian application no. 202221068002, filed on November 25, 2022.

TECHNICAL FIELD

The disclosure herein generally relates to spiking neural networks, and, more particularly, to method and system for spiking neural network-based ECG classifier for wearable edge devices.

BACKGROUND

Electrocardiogram (ECG) is one of the most extensively employed signals used to diagnose and predict cardiovascular diseases (CVDs) which provides opportunities for earlier prevention and intervention. In recent years, personal wearable devices have been introduced as cost-effective solutions for continuous ECG monitoring in daily life. ECG signal recorded by wearable electronic devices is with low-frequency and low-amplitude and is inevitably contaminated by a variety of noises and artifacts. Classical algorithms are mainly based on morphological features, such as the QRS complex and signal processing techniques such as Fourier transform, and wavelet transform. Visual inspection of recorded ECG signals during a visit to the cardiologist is the traditional form of arrhythmia detection. However, due to their intermittent occurrence specially in early stages of the problem, arrhythmias are difficult to detect from a short time window of the ECG signal. Therefore, continuous ECG monitoring is crucial in early detection of potential problems.
Neural network algorithms automatically extract the features from ECG signals and hence, provide higher accuracy. This is because neural network based feature extraction is inherently more resilient to variations among different ECG waveforms However, employing deep neural networks have high computational intensity and large power consumption. In last few years, several deep learning based ECG classification models have been developed that can classify ECG signals with higher accuracy based on features like heart rhythms. However, these deep learning based ECG classification models have computational complexity, memory, and power requirement. Existing deep learning based classification models lack in executing low-power, low-memory, and battery driven devices. In an effort to continuously monitor and detect abnormalities in patients ECG signals on portable devices, ECG heartbeat classification method based on a spiking neural network (SNN) is required. Spiking neural networks (SNN) are the third generation of neural networks and provide the opportunity for ultra-low power operation. In this type of neural networks, the information is processed based on propagation of spike signals through the network and the timing of the spikes. Every neuron consumes energy only when it sends or receives spikes.

SUMMARY

Embodiments of the present disclosure present technological improvements as solutions to one or more of the above-mentioned technical problems recognized by the inventors in conventional systems. For example, in one embodiment, a method for spiking neural network based ECG classifier for wearable edge devices is provided. The system includes a spiking neural network which receives a training dataset comprising a plurality of ECG time-series data from a plurality of sensors. The spiking neural network comprise at least one of a first spiking neural network and a second spiking neural network. The first spiking neural network comprise a reservoir-based spiking neural network and a feed forward based spiking neural network. Each of the spiking neural network comprise a logistic regression-based ECG classifier which is being trained to classify one or more class labels. The peak-based spike encoder of each spiking neural network obtains a plurality of encoded spike trains based on one or more peaks detected from each ECG time-series data. Further, the plurality of encoded spike trains are provided into at least one of the spiking neural network. Further, a reservoir of the first spiking neural network obtains neuronal trace values of a plurality of recurrently connected excitatory neurons for the plurality of encoded spike trains. Then, a spiking neural network layer (SNN) layer of the second spiking neural network obtains neuronal trace values of a plurality of neurons connected in a feed forward pattern. Further, a first set of spatio-temporal features are extracted for each ECG time-series data from the first spiking neural network based on the neuronal trace values of the plurality of excitatory neurons. Then, a second set of spatio-temporal features are extracted from the second spiking neural network based on the neuronal trace values of the plurality of feed forward neurons. The logistic regression-based ECG classifier of each of the spiking neural network are trained with the neuronal trace values of the plurality of recurrently connected excitatory neurons and the neuronal trace values of the plurality of neurons connected in the feed forward pattern to classify one or more class labels. Furthermore, a plurality of test dataset are received as input into at least one of the spiking neural network to obtain associated class label from the logistic regression-based ECG classifier.

In another aspect, a method for spiking neural network based ECG classifier for wearable edge devices is provided. The method includes a spiking neural network which receives a training dataset comprising a plurality of ECG time-series data from a plurality of sensors. The spiking neural network comprise at least one of a first spiking neural network and a second spiking neural network. The spiking neural network comprise a reservoir-based spiking neural network and a feed forward based spiking neural network. Each of the spiking neural network comprise a logistic regression-based ECG classifier which is being trained to classify one or more class labels. The peak-based spike encoder of each spiking neural network obtains a plurality of encoded spike trains based on one or more peaks detected from each ECG time-series data. Further, the plurality of encoded spike trains are provided into at least one of the spiking neural network. Further, a reservoir of the first spiking neural network obtains neuronal trace values of a plurality of recurrently connected excitatory neurons for the plurality of encoded spike trains. Then, a spiking neural network layer (SNN) layer of the second spiking neural network obtains neuronal trace values of a plurality of neurons connected in a feed forward pattern. Further, a first set of spatio-temporal features are extracted for each ECG time-series data from the first spiking neural network based on the neuronal trace values of the plurality of excitatory neurons. Then, a second set of spatio-temporal features are extracted from the second spiking neural network based on the neuronal trace values of the plurality of feed forward neurons. The logistic regression-based ECG classifier of each of the spiking neural network are trained with the neuronal trace values of the plurality of recurrently connected excitatory neurons and the neuronal trace values of the plurality of neurons connected in the feed forward pattern to classify one or more class labels. Furthermore, a plurality of test dataset are received as input into at least one of the spiking neural network to obtain associated class label from the logistic regression-based ECG classifier.

In yet another aspect, a non-transitory computer readable medium for the system includes a spiking neural network which receives a training dataset comprising a plurality of ECG time-series data from a plurality of sensors. The spiking neural network comprise at least one of a first spiking neural network and a second spiking neural network. The spiking neural network comprise a reservoir-based spiking neural network and a feed forward based spiking neural network. Each of the spiking neural network comprise a logistic regression-based ECG classifier which is being trained to classify one or more class labels. The peak-based spike encoder of each spiking neural network obtains a plurality of encoded spike trains based on one or more peaks detected from each ECG time-series data. Further, the plurality of encoded spike trains are provided into at least one of the spiking neural network. Further, a reservoir of the first spiking neural network obtains neuronal trace values of a plurality of recurrently connected excitatory neurons for the plurality of encoded spike trains. Then, a spiking neural network layer (SNN) layer of the second spiking neural network obtains neuronal trace values of a plurality of neurons connected in a feed forward pattern. Further, a first set of spatio-temporal features are extracted for each ECG time-series data from the first spiking neural network based on the neuronal trace values of the plurality of excitatory neurons. Then, a second set of spatio-temporal features are extracted from the second spiking neural network based on the neuronal trace values of the plurality of feed forward neurons. The logistic regression-based ECG classifier of each of the spiking neural network are trained with the neuronal trace values of the plurality of recurrently connected excitatory neurons and the neuronal trace values of the plurality of neurons connected in the feed forward pattern to classify one or more class labels. Furthermore, a plurality of test dataset are received as input into at least one of the spiking neural network to obtain associated class label from the logistic regression-based ECG classifier.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for ECG time-series data classifier using a spiking neural network, in accordance with some embodiments of the present disclosure.
FIG.2 is an exemplary block diagram showing an architecture of a first spiking neural network, in accordance with some embodiments of the present disclosure.
FIG.3 is an exemplary block diagram showing an architecture of a second spiking neural network, in accordance with some embodiments of the present disclosure.
FIG.4 illustrates an exemplary flow diagram of a method for ECG time-series data classification using the spiking neural network, in accordance with some embodiments of the present disclosure.
FIG.5 through FIG.8 shows a performance comparison of a peak-based spike encoder versus a Gaussian encoder and a Delta modulator on a sample ECG time-series data, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

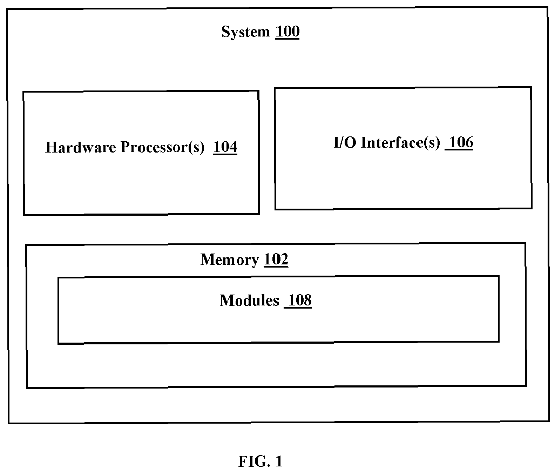

Referring now to the drawings, and more particularly to FIG. 1 through FIG.8, where similar reference characters denote corresponding features consistently throughout the figures, there are shown preferred embodiments and these embodiments are described in the context of the following exemplary system and/or method.

Embodiments herein provide a method and system for spiking neural network based ECG classifier for wearable edge devices. The system may be alternatively referred as ECG time-series data classifier system 100. Real-time monitoring of ECG signal at wearable and implantable devices for example smart watch, pacemaker and the like are crucial for early clinical intervention of cardiovascular diseases. In view of the foregoing challenges spiking neural network coupled with non-von Neumann Neuromorphic Computing (NC) platforms provides ECG time-series data classification at edge. The input ECG time-series data is first segmented into heartbeats. The heartbeat is split into a small number of windows, which are then encoded into spikes. The patterns in the generated spikes are automatically extracted by using one or more spiking neural network (SNN) architectures. The method of the present disclosure is implemented with two spiking neural network (SNN) architectures, where a first spiking neural network comprise a reservoir-based spiking neural network and a second spiking neural network comprise a feed-forward based spiking neural network. The SNNs are created using a Leaky-Integrate and Fire (LIF) neuron model as it is computationally easier to simulate. The first spiking neural network and the second spiking neural network comprise a peak-based spike encoder which improves performance with least computational effort and minimized power consumption. The disclosed system is further explained with the method as described in conjunction with FIG. 1 to FIG.8 below.

FIG. 1 illustrates an exemplary block diagram of a system for ECG time-series data classifier using a spiking neural network, in accordance with some embodiments of the present disclosure. In an embodiment, the batch processing system 100 includes processor (s) 104, communication interface (s), alternatively referred as or input/output (I/O) interface(s) 106, and one or more data storage devices or memory 102 operatively coupled to the processor (s) 104. The system 100, with the processor(s) is configured to execute functions of one or more functional blocks of the system 100.

Referring to the components of the system 100, in an embodiment, the processor (s) 104 can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the processor(s) 104 is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

The I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices (nodes) of the system 100 to one another or to another server.

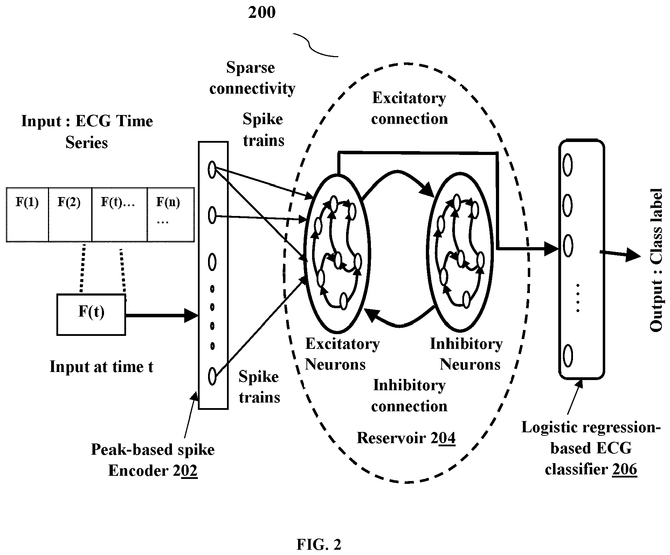

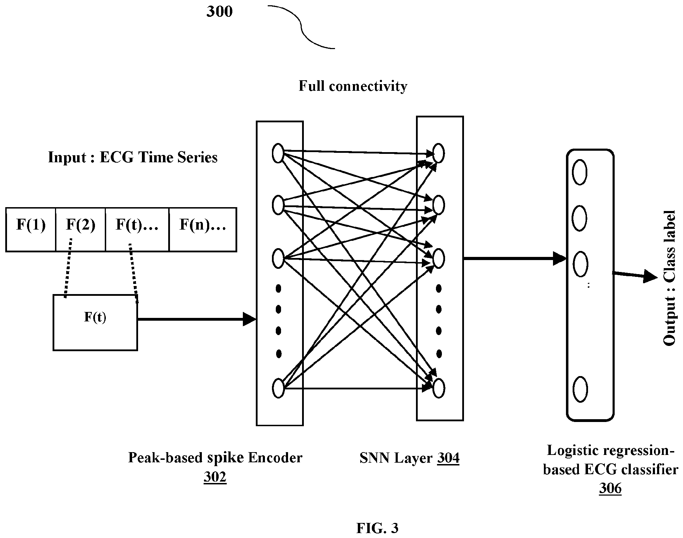

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. The memory 102 may comprise information pertaining to input(s)/output(s) of each step performed by the processor(s) 104 of the system 100 and methods of the present disclosure. Functions of the components of system 100, for predicting batch processes, are explained in conjunction with FIG.2 and FIG.3 providing flow diagram, architectural overviews, and performance analysis of the system 100.

FIG.2 is an exemplary block diagram showing an architecture of a reservoir-based spiking neural network, in accordance with some embodiments of the present disclosure. The reservoir-based spiking neural network 200 comprise a peak-based spike encoder 202, a reservoir 204, and a logistic regression based classifier 206.

In one example a wearable ECG device communicates for example by Bluetooth to a mobile device (e.g., a mobile phone) carrying an application that buffers and uploads the data to a server through mobile or Wi-Fi networks. The reservoir-based spiking neural network 200 processes the training data comprising a plurality of ECG time-series data.

The peak-based spike encoder 202 generates a plurality of encoded spike trains for the plurality of ECG time-series data.

The reservoir 204 processes the plurality of encoded spike trains to obtain the neuronal trace values. The one or more connections between the peak-based spike encoder 202 and the reservoir is sparse. The reservoir 204 is a recurrently connected population of excitatory and inhibitory neurons. The connectivity between neurons are sparse, probabilistic and remain within the same population so that dynamical stability of the network is ensured. This neuronal population is very efficient in extracting one or more spatio-temporal features from ECG time-series data. Recurrent weights with directed cycles act as a non-linear random projection of the sparse input feature space to a higher dimensional spatio-temporal embedding, thereby generating explicit temporal features. These embeddings are captured in the form of neuronal traces of the reservoir neurons.

The logistic regression based ECG classifier 206 of the system 200 is trained to determine corresponding class label by passing neuronal trace values of a plurality of recurrently connected excitatory neurons.

FIG.3 is an exemplary block diagram showing an architecture of a second spiking neural network, in accordance with some embodiments of the present disclosure. In another embodiment, the feed forward based spiking neural network 200 comprise a peak-based spike encoder 302, a spiking neural network (SNN) layer 304, and a logistic regression-based ECG classifier 306. Once the input spike train is fed into the feed forward based spiking network 300, these connections learn via spike timing dependent plasticity (STDP) rule. Post training, the feed forward based spiking neural network 300 captures the learned features (aka corresponding neuronal activities) in neuronal trace values.

The peak-based spike encoder 302 generates the plurality of encoded spike trains for the plurality of ECG time-series data.

The spiking neural network (SNN) layer 304 processes the plurality of encoded spike trains to obtain neuronal trace values. The SNN layer is a simple network topology where the neurons are connected across consecutive layers but not within same layer where full connectivity is used.

The logistic regression-based ECG classifier 306 is trained to determine corresponding class label for the neuronal trace values of a plurality of feed forward neurons.

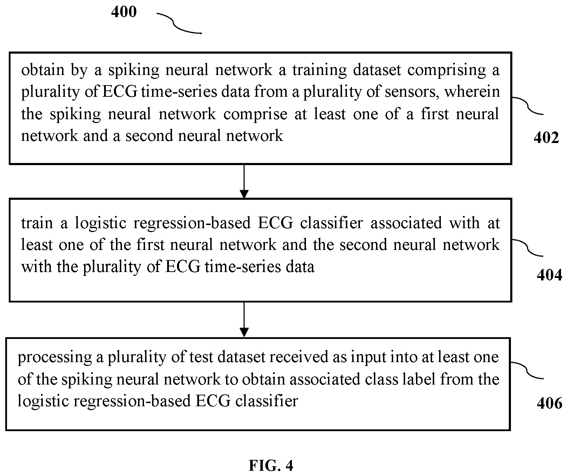

FIG.4 illustrates an exemplary flow diagram of a processor implemented method for ECG time-series data classification using the spiking neural network, in accordance with some embodiments of the present disclosure. In an embodiment, the system 100 comprises one or more data storage devices or the memory 102 operatively coupled to the processor(s) 104 and is configured to store instructions for execution of steps of the method 300 by the processor(s) or one or more hardware processors 104. The steps of the method 300 of the present disclosure will now be explained with reference to the components or blocks of the system 100 as depicted in FIG. 1 through FIG.3 and the steps of flow diagram as depicted in FIG.4. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring now to the steps of the method 400, at step 402, the one or more hardware processors 104 obtain by a spiking neural network a training dataset comprising a plurality of ECG time-series data from a plurality of sensors, wherein the spiking neural network comprise at least one of a first spiking neural network and a second spiking neural network. Spiking neural network is a third generation of neural networks, referred to as SNNs, provides a low-power operation. In this type of network, the information is processed based on the propagation of spike signals through the network and the timing of the spikes. Dense network of neurons are interconnected which generates spikes from one neuron to other interconnected neuron. The first spiking neural network may be for example a reservoir-based spiking neural network 200 and a second spiking neural network may be for example a feed-forward spiking neural network 300. It is to be noted that the spiking neural networks are not limited to the reservoir-based spiking neural network and the feed-forward spiking neural network. In specific, neurons communicate through electrical signals called spikes. For example, a neuron $i$ fires at time $t$ which generates a spike on its output at time $t$, only when its membrane potential reaches a specific threshold value. The information is encoded in the timing of the spikes, not in their amplitude.

The training dataset comprise the plurality of ECG time-series data is the input to the reservoir-based spiking neural network 200. The training dataset may include for example ECG Atrial Fibrillation datasets with different attributes and university of California riverside (UCR) datasets namely ECG200, ECG5000 and the like. The plurality of ECG time-series data is represented as $\{F(1), F(2),..F(n)\}$. Here, at time t, where F(t) is being one sample value in the time series and is further fetched by the peak-based spike encoder 202. The plurality of ECG time-series data are processed to obtains the neuronal trace values of a plurality of recurrently connected excitatory neurons. The peak-based spike encoder 202 processes the plurality of ECG time-series data to obtain a plurality of encoded spike trains based on one or more peaks.

The reservoir 204 obtains the plurality of encoded spike trains from the peak-based spike encoder 202 and obtains neuronal trace values of a plurality of recurrently connected excitatory neurons. Further, a first set of spatio-temporal features are extracted for each ECG time-series data based on the neuronal trace values of the plurality of excitatory neurons.

The logistic regression-based ECG classifier 206 of the reservoir-based spiking neural network 200 is trained by passing the neuronal trace values of the plurality of recurrently connected excitatory neurons to learn one or more class labels.

Similarly, the feed-forward spiking neural network 300 also fetches the training dataset as input. Here, the training dataset comprise the plurality of ECG time-series data is represented as {$F(1)$, $F(2)$,..$F(n)$} at time $F(t)$. Here, at time t, where $F(t)$ is being one sample value in the time series and is further fetched by the peak-based spike encoder 302. The plurality of ECG time-series data are processed to obtain neuronal trace values of a plurality of feed forward neurons. The peak-based spike encoder 302 processes the plurality of ECG time-series data to obtain a plurality of encoded spike trains based on one or more peaks detected.

The SNN layer 304 obtains neuronal trace values of a plurality of feed forward neurons from the plurality of encoded spike trains. Further, a second set of spatio-temporal features are extracted based on the neuronal trace values of the plurality of feed forward neurons for each ECG time-series data from each feed-forward neuron.

The logistic regression-based ECG classifier 306 is trained by a plurality of encoded spike trains based on one or more peaks detected from each ECG time-series data.

Referring now to the steps of the method 400, at step 404, the one or more hardware processors 104 train a logistic regression-based ECG classifier associated of at least one of the first spiking neural network and the second spiking neural network with the plurality of ECG time-series data. The peak-based spike encoder 202 of the reservoir-based spiking neural network 200 and the peak-based spike encoder 302 of the feed-forward spiking neural network 300 process the plurality of ECG time-series data to obtain the plurality of encoded spike trains by initially filtering the plurality of ECG time-series data by using a low pass filter.

Further, the plurality of ECG time-series data are passed through an integrate and fire (IF) neuron model having an initial membrane threshold value. Further, the method detects one or more spike trains of the plurality of ECG time-series data which occurred above a first threshold timestep and discards one or more spike trains that precede below the first threshold timestep. The first threshold timestep may be a value 200. Such example of threshold timestep shall not be construed as limiting the scope of the present disclosure. The one or more spike trains of each ECG time-series data comprising a positive slope and a negative slope are identified.

Further, the one or more spike trains of each ECG time-series data are classified as a first wave if the occurrence of each spike train is within a second threshold timestep and if maximum derivative of each ECG time-series data is lesser than half the maximum derivative of each spike train occurred at the time of previous instance. The first wave may be for example a T-wave. The second threshold timestep may be a value 360.

Further, the one or more spike trains of each ECG time-series data are classified as a second wave if the occurrence of each spike train is above the second threshold timestep and if maximum derivative of each ECG time-series data is greater than half the maximum derivative of each spike train occurred at the time of previous instance and if one or more spiking activities of each spike train exceeds a third threshold and otherwise classified as noise Further, the initial membrane threshold value of the IF neuron model are adjusted based on the change occurred in the plurality of ECG time-series data and a previous spiking activity. The second wave may be for example a QRS-complex. The peaks, onsets, and offsets of ECG components (for example, QRS complex and T-wave) are detected and their precise timings are taken as spike event times. For each of these components separate spike trains are obtained but the temporal correlation is maintained in the spike domain.

Referring now to the steps of the method 400, at step 406 process a plurality of test dataset received as input into at least one of the spiking neural network to obtain associated class label from the logistic regression-based ECG classifier. The plurality of test dataset are first encoded into one or more spike trains. Then each spike train is passed into at least one of the spiking neural network for feature extraction and learning. Neuronal activities are then presented to a simple linear classifier for final classification.

The stability and performance (referring to Table 1) of each spiking neural network depend heavily on careful tuning of different network parameters. For the reservoir-based spiking neural network 200 the major parameters are tuned with number of excitatory and inhibitory neurons ($N_{ex}$ and $N_{inhi}$ respectively) , Number of recurrent connections $N_{rec}$ various connectivity parameters like input encoder outdegree ($Input_{outdegree}$) which controls the connection between the spike encoder and the reservoir. Moreover, a 5-tuple of weight scalar values of, (*input - to - excitatory, excitatory - to - input, inhibitory - to - excitatory, inihibitory - to - inihibitory*) are tuned and fixed for the inter-population network connections having $N_{rec} = \frac{N_{ex}}{3}$

Table 1 - Network Parameters

| Dataset | ECG200 | ECG50 00 | ECGfiveday | PhysioNet2 017 | MIT-BIH |
|---|---|---|---|---|---|
| $N_{ex}$ | 2000 | 3000 | 3000 | 4000 | 4000 |

(continued)

| Dataset | ECG200 | ECG50 00 | ECGfiveday | PhysioNet2 017 | MIT-BIH |
|---|---|---|---|---|---|
| $N_{inhi}$ | 1000 | 1000 | 500 | 1000 | 1000 |
| $Input_{outdegree}$ | 500 | 500 | 300 | 500 | 500 |
| $Weightscalar$ | (2.0,0.7,1 .8,0.3,0.9 ) | (2.0,0.7, 0.3,1.1) | (2.0,1.5,0.7, 0.5,1.5) | (2.0,1.0,0.1, 0.9,1.7) | (2.0,1.7, 0.4,0.8,1 .2) |
| $T_m$ | 20 | 25 | 20 | 30 | 25 |
| Delta Modulator + Reservoir | | | | | |
| $N_{ex}$ | 3000 | 2000 | 2000 | 4000 | 2000 |
| $N_{inhi}$ | 1000 | 1000 | 500 | 1000 | 1000 |
| $Input_{outdegree}$ | 500 | 300 | 300 | 300 | 300 |
| $Weightscalar$ | (2.0,1.6,0 .9,0.6,1.4 ) | (2.0,1.1, 0.3,0.9, 1.2) | (2.0,0.9,0.1, 0.5,1.8) | (2.0,1.9,0.1, 0.3,1.2) | (2.0,1.2, 0.4,0.3,0 .8) |
| $T_m$ | 20 | 30 | 20 | 30 | 25 |
| Peak-based spike encoder + Reservoir | | | | | |
| $N_{ex}$ | 2000 | 2000 | 2000 | 3000 | 2000 |
| $N_{inhi}$ | 500 | 1000 | 1000 | 1000 | 1000 |
| $Input_{outdegree}$ | 300 | 500 | | 300 | 500 |
| $Weightscalar$ | (2.0,0.6,1 .9,0.5,1.8 ) | (2.0,1.7, 0.9,0.1, 1.4) | (2.0,0.3,1.1, 0.9,1.1) | (2.0,1.9,1.3, 1.1,1.9) | (2.0,1.5, 0.3,0.2,0 .9) |
| $T_m$ | 30 | 35 | 25 | 30 | 25 |
| Peak-based spike encoder | | | | | |
| $N_{FF}$ | 1000 | 1000 | 2000 | 2000 | 1000 |
| $T_m$ | 30 | 25 | 20 | 35 | 30 |

The feed-forward based spiking neural network 300 has number of neurons in the SNN layer. For both the spiking? neural network architectures, the threshold voltage = -55.0 and resting potential = -65.0 for IF neuron model. The membrane time constant (m) of LIF controls the decay of the neuronal membrane 291 potential and has been set to different values for different cases.

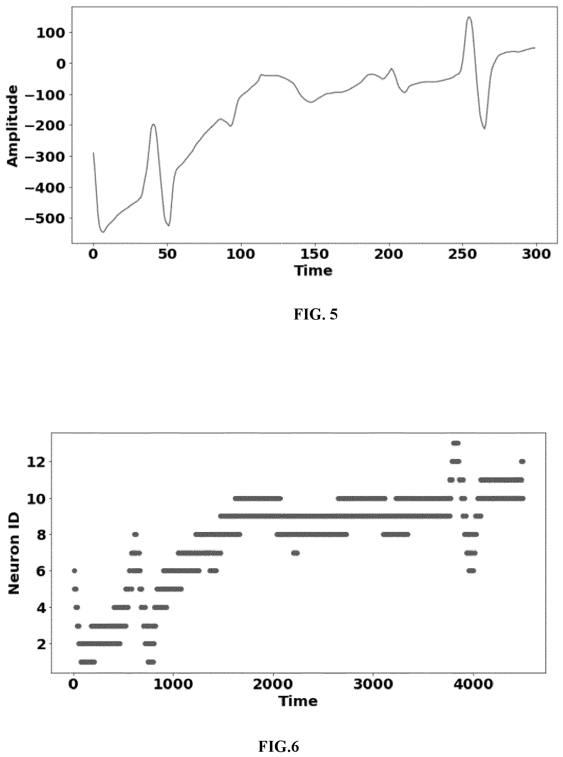

FIG.5 through FIG.8 shows a performance comparison of a peak-based spike encoder versus a Gaussian encoder and a Delta modulator on a sample ECG time-series data, in accordance with some embodiments of the present disclosure. FIG.5 shows graphical representation of sample ECG time-series data having a small segment of an ECG signal of length 300 timesteps.

FIG.6 shows graphical representation of a plurality of spike trains generated for the ECG time-series data using a Gaussian encoder representing the original ECG time-series data as a dense spike train (for example 15 times 80 magnified in time scale) resulting in a greater number of computations and processing time. The Gaussian encoder have a neuron-population based temporal encoding scheme which is frequently used due to its ability to capture granular details of the input test dataset. Here, multiple neurons encode different segments of the input range. It encodes one input-value into a time magnified spike train.

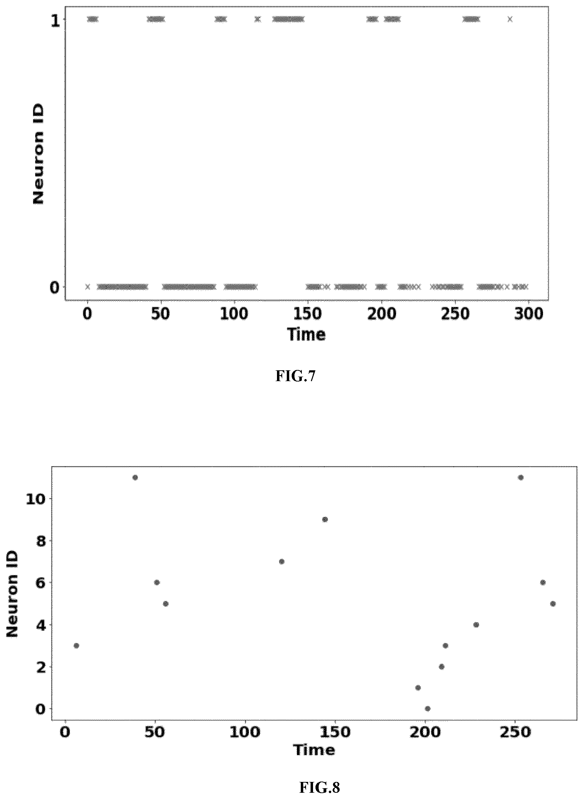

FIG.7 shows graphical representation of a plurality of spike trains generated for the ECG time-series data using the Delta modulator which encodes the ECG signal into two representative ON/OFF spike trains. Delta modulator is an ECG specific spike encoder where the change in value of ECG signal between two consecutive timesteps is encoded as spike events (ON or OFF based on positive or negative change) if the difference is above a certain threshold. Threshold determines the sparsity and pattern of encoded spike train. The on-demand nature of the encoding (for example, if input signal is not changing, no output spikes are produced) increases its efficiency.

FIG.8 shows graphical representation of a plurality of spike trains generated for the ECG time-series data using a peak-based spike encoder, in accordance with some embodiments of the present disclosure. The peak-based spike encoder processes only the peaks and waves which are relevant for understanding the ECG signature. As a result, the number of spikes are extremely low and sparsely distributed in the time axis. While spike trains from both Gaussian encoder and the Delta modulator encoder have more granular information about the ECG signal, they may also include redundant noise that might hamper the final classification task. However, Peak encoder stresses upon ECG signal morphology instead of concentrating each and every value in the signal.

In one embodiment, the system 100 is validated with ECG Atrial Fibrillation datasets with different attributes and complexity. The method of the present disclosure was initially validated with UCR datasets such as ECG200, ECG5000, and the like. Each of the datasets contained ECG time-series data of people from different age group, gender recorded over different time period and having distinctive features. It is noted that repository of freely available medical research data (PhysioNet) dataset provides ECG recording (between 30 sec and 60 sec in length), where the recording shows 4 classes for example normal sinus rhythm, atrial fibrillation (AF), an alternative rhythm, or is too noisy to be classified. The last one is widely known Massachusetts Institute of Technology arrhythmia database(MIT-BIH) having 48 half hour long two-channel ECG recordings of 47 participants.

In one embodiment, the method of the present disclosure is implemented using a graphical processing unit (GPU) based SNN simulator. Here, for each of the datasets, both the spiking neural networks are tuned with parameters such as number of neurons and its associated connections, and neuronal model parameters obtains the best performance. The details of the SNN hyper-parameters are discussed in later embodiments.

The reservoir-based spiking neural network 200 understands the dynamics of temporal varying ECG time-series data. However, the classification accuracy of the reservoir 204 is observed to be lower than that of existing deep learning as referred in Table 2. Gaussian encoder magnifies the whole ECG time-series data in temporal spike domain when focused on specific ECG wave signatures.

## Table 2 – Classification accuracy for different ECG dataset

| Dataset | Train size | Length | Number of classes | Test size | SOA accuracy % | Gaussian encoder + Reservoir accuracy % | Delta modulator + Reservoir accuracy % | Peak-based spike encoder + Reservoir accuracy % | Peak-based spike encoder + feed-forward accuracy % |
|---|---|---|---|---|---|---|---|---|---|
| ECG200 | 100 | 96 | 2 | 100 | 89[26] | 81 | 84 | 83 | 81 |
| ECG5000 | 500 | 140 | 5 | 4500 | 99[30] | 89.71 | 91.7 | 93.1 | 92 |
| ECG fivedays | 23 | 136 | 2 | 861 | 98.2[4] | 80 | 82.7 | 86.6 | 88.9 |
| PhysioNet2017 | 6822 | 2k to 9k | 4 | 1705 | 83[10] | 69 | 72 | 78.5 | 77 |
| MIT-BIH | 13K | 3K | 4 | 3.1K | 95.6[9] | 91.6 | 94.1 | 93.5 | 94.3 |

As a result, one or more spikes enters into the first spiking neural network 200 and the neuronal activities of the reservoir 204 starts overlapping different classes thereby reducing the accuracy. The Delta modulator encoder improves the accuracy of the reservoir 204 on-demand encoding and resulting sparsity in spikes. However, when the method of the present disclosure the peak-based spike encoder 202 when coupled with the reservoir-based spiking neural network 200 achieves better accuracy when compared to other two existing encoders.

Upon replacing the reservoir 204 with a computationally light feed-forward spiking network 300 (with peak-based spike encoder), the accuracy does not degrade much and improves the performance. It is observed that the peak-based spike encoder 202 achieves highest accuracy across all five datasets having variety in terms of size of training dataset and testing dataset, data complexity, length of each sample ECG time series and number of classes. Also, it has been experimentally noted that if the feed-forward based spiking neural network 300 is evaluated on PhysioNet data classifies normal and abnormal AF classes only, then it has achieved with 92% accuracy. Performance of peak based spike encoder is superlative because of efficient ECG morphology specific spike encoding.

Further, usually the number of synaptic operations is considered as the average number of spikes per timestep for the entire training and inference. Here, for all the network encoder combinations and for all the five datasets, the synaptic operations per timestep indicates an estimated energy consumption on NC platforms. Table 3 shows the best estimates for number of synaptic operations per timestep.

Table 3 -Synaptic operations comparison of different architectures

| Dataset | Gaussian encoder + Reservoir time step (ts) | Delta modulator + Reservoir time step (ts) | Peak-based spike encoder +Reservoir time step (ts) | Peak-based spike encoder +Feed-forward network time step (ts) |
|---|---|---|---|---|
| ECG200 | 19/ts | 21/ts | 21/ts | 18/ts |
| ECG5000 | 29/ts | 26/ts | 24/ts | 24/ts |
| ECG five days | 23/ts | 20/ts | 22/ts | 17/ts |
| PhysioNet 2017 | 32/ts | 36/ts | 32/ts | 28/ts |
| MIT-BIH | 45/ts | 41/ts | 37/ts | 33/ts |

As expected, owing to large spike activity, Gaussian encoder with reservoir is computationally most expensive whereas peak-based spike encoder with the feed forward based spiking neural network has sparse spikes encoded and a smaller number of neurons in network.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of spiking neural networks. The embodiment, thus provides method and system for spiking neural network based ECG classifier for wearable edge devices. Moreover, the embodiments herein further provide the peak-based spike encoder helps achieving highest performance for classifying labels. However, the real benefit comes when computational cost and energy consumption is looked into. Energy requirement of an SNN is directly proportional to total number of synaptic operations (SOP) executed during runtime. Encoding performance of peak-based spike encoder can be improved by removing the noise peaks in ECG time-series data by correlating the signal from another auxiliary low energy sensor and the like.

It is to be understood that the scope of the protection is extended to such a program and in addition to a computer-readable means having a message therein such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components.

For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (400) of a spiking neural network-based Electrocardiogram (ECG) classifier for wearable edge devices comprising:

obtaining (402) by a spiking neural network via one or more hardware processors, a training dataset comprising a plurality of ECG time-series data from a plurality of sensors, wherein the spiking neural network comprise at least one of a first spiking neural network and a second spiking neural network;
training (404) via the one or more hardware processors, at least one of the first spiking neural network and the second spiking neural network with the plurality of ECG time-series data to obtain a logistic regression-based ECG classifier, wherein the training comprises:

feeding the plurality of ECG time-series data into a peak-based spike encoder of at least one of the spiking neural network to obtain a plurality of encoded spike trains from each spiking neural network based on one or more peaks detected from each ECG time-series data;
providing the plurality of encoded spike trains into at least one of the spiking neural network, wherein a reservoir of the first spiking neural network obtains neuronal trace values of a plurality of recurrently connected excitatory neurons, and wherein a spiking neural network (SNN) layer of the second spiking neural network obtains neuronal trace values of a plurality of neurons connected in a feed forward pattern;
extracting from each ECG time-series data, a first set of spatio-temporal features from the first spiking neural network based on the neuronal trace values of the plurality of excitatory neurons, and a second set of spatio-temporal features from the second spiking neural network based on the neuronal trace values of the plurality of neurons connected in the feed forward pattern;
training the logistic regression-based ECG classifier with one or more class labels associated with each of the spiking neural network by passing corresponding neuronal trace values of the plurality of recurrently connected excitatory neurons into the first neural network and the neuronal trace values of the plurality of neurons connected in the feed forward pattern into the second neural network; and

processing (406) via the one or more hardware processors, a plurality of test dataset received as input into at least one of the spiking neural network to obtain associated class label from the logistic regression-based ECG classifier.

**2.** The processor implemented method as claimed in claim 1, wherein the peak-based spike encoder of at least one of the spiking neural network obtains the plurality of encoded spike trains by performing the steps of:

filtering the plurality of ECG time-series data by using a low pass filter;

passing the plurality of ECG time-series data through an integrate and fire (IF) neuron model having an initial membrane threshold value;

detecting one or more spike trains of the plurality of ECG time-series data which occurred above a first threshold timestep and discarding one or more spike trains that precede below the first threshold timestep;

identifying if the one or more spike trains of each ECG time-series data comprise a positive slope and a negative slope;

classifying the one or more spike trains of each ECG time-series data as a first wave, if the occurrence of each spike train is within a second threshold timestep and if maximum derivative of each ECG time-series data is lesser than half the maximum derivative of each spike train occurred at the time of previous instance;

classifying the one or more spike trains of each ECG time-series data as a second wave, if the occurrence of each spike train is above the second threshold timestep and if maximum derivative of each ECG time-series data is greater than half the maximum derivative of each spike train occurred at the time of previous instance and if one or more spiking activities of each spike train exceeds a third threshold and otherwise classified as noise; and

adjusting the initial membrane threshold value of the IF neuron model based on the change occurred in the plurality of ECG time-series data and previous spiking activity.

**3.** The processor implemented method as claimed in claim 1, wherein the first spiking neural network comprise a reservoir-based spiking neural network and the second spiking neural network comprise a feed forward based spiking neural network.

**4.** The processor implemented method as claimed in claim 1, wherein the plurality of spike trains are sparsely distributed in time axis.

**5.** The processor implemented method as claimed in claim 1, wherein the first wave includes a T-wave, and the second wave includes a QRS complex.

**6.** The processor implemented method as claimed in claim 1, wherein the recurrent connections between neurons of the first spiking neural network are sparse and probabilistic which provides dynamic stability.

**7.** The processor implemented method as claimed in claim 1, wherein the first spiking neural network comprises the peak-based spike encoder, a reservoir, and the logistic regression based ECG classifier, and wherein one or more connections between the peak-based spike encoder and the reservoir is sparse.

**8.** The processor implemented method as claimed in claim 1, wherein the second spiking neural network comprise the peak-based spike encoder, the feed forward based spiking neural network (SNN) layer, and the logistic regression based ECG classifier, and wherein one or more connections between the peak-based spike encoder and the spiking neural network is fully connected.

**7.** A system (100) of a spiking neural network based Electrocardiogram (ECG) classifier for wearable edge devices comprising:

a memory (102) storing instructions;

one or more communication interfaces (106); and

one or more hardware processors (104) coupled to the memory (102) via the one or more communication interfaces (106), wherein the one or more hardware processors (104) are configured by the instructions to:

obtaining by a spiking neural network a training dataset comprising a plurality of ECG time-series data from a plurality of sensors, wherein the spiking neural network comprise at least one of a first spiking neural network and a second spiking neural network;

training at least one of the first spiking neural network and the second spiking neural network with the plurality of ECG time-series data to obtain a logistic regression-based ECG classifier, wherein the training comprises:

feeding the plurality of ECG time-series data into a peak-based spike encoder of at least one of the spiking neural network to obtain a plurality of encoded spike trains from each spiking neural network based on one or more peaks detected from each ECG time-series data;

providing the plurality of encoded spike trains into at least one of the spiking neural network, wherein a reservoir of the first spiking neural network obtains neuronal trace values of a plurality of recurrently connected excitatory neurons, wherein a spiking neural network layer (SNN) of the second spiking neural network obtains neuronal trace values of a plurality of neurons connected in a feed forward pattern;

extracting from each ECG time-series data, a first set of spatio-temporal features are extracted from the first spiking neural network based on the neuronal trace values of the plurality of excitatory neurons, and a second set of spatio-temporal features are extracted from the second spiking neural network based on the neuronal trace values of the plurality of neurons connected in the feed forward pattern; training the logistic regression-based ECG classifier with one or more class labels associated with each of the spiking neural network by passing the neuronal trace values of the plurality of recurrently connected excitatory neurons into the first neural network and the neuronal trace values of the plurality of neurons connected in the feed forward pattern into the second neural network; and

processing a plurality of test dataset received as input into at least one of the spiking neural network to obtain associated class label from the logistic regression-based ECG classifier.

**8.** The system of claim 7, wherein the peak-based spike encoder of at least one of the spiking neural network obtains the plurality of encoded spike trains by performing the steps of:

filtering the plurality of ECG time-series data by using a low pass filter;
passing the plurality of ECG time-series data through an integrate and fire (IF) neuron model having an initial membrane threshold value;
detecting one or more spike trains of the plurality of ECG time-series data which occurred above a first threshold timestep and discarding one or more spike trains that precede below the first threshold timestep;
identifying if the one or more spike trains of each ECG time-series data comprise a positive slope and a negative slope;
classifying the one or more spike trains of each ECG time-series data as a first wave, if the occurrence of each spike train is within a second threshold timestep and if maximum derivative of each ECG time-series data is lesser than half the maximum derivative of each spike train occurred at the time of previous instance;
classifying the one or more spike trains of each ECG time-series data as a second wave, if the occurrence of each spike train is above the second threshold timestep and if maximum derivative of each ECG time-series data is greater than half the maximum derivative of each spike train occurred at the time of previous instance and if one or more spiking activities of each spike train exceeds a third threshold and otherwise classified as noise; and
adjusting the initial membrane threshold value of the IF neuron model based on the change occurred in the plurality of ECG time-series data and previous spiking activity.

**9.** The system of claim 7, wherein the first spiking neural network comprise a reservoir-based spiking neural network and the second spiking neural network comprise a feed forward based spiking neural network,

wherein the first spiking neural network comprises the peak-based spike encoder, a reservoir, and the logistic regression-based ECG classifier, and wherein one or more connections between the peak-based spike encoder and the reservoir is sparse, and
wherein the second spiking neural network comprise the peak-based spike encoder, a spiking neural network layer, and the logistic regression-based ECG classifier, and wherein one or more connections between the peak-based spike encoder and the spiking neural network is fully connected.

**10.** The system of claim 7, wherein the plurality of spike trains are sparsely distributed in time axis.

**11.** The system of claim 7, wherein the first wave includes a T-wave, and the second wave includes a QRS complex.

**12.** The system of claim 7, wherein the recurrent connections between neurons of the first spiking neural network are sparse and probabilistic which provides dynamic stability.

**13.** One or more non-transitory machine-readable information storage mediums comprising one or more instructions which when executed by one or more hardware processors cause:

obtaining by a spiking neural network a training dataset comprising a plurality of ECG time-series data from a plurality of sensors, wherein the spiking neural network comprise at least one of a first spiking neural network and a second spiking neural network;

training at least one of the first spiking neural network and the second spiking neural network with the plurality of ECG time-series data to obtain a logistic regression-based ECG classifier, wherein the training comprises:

feeding the plurality of ECG time-series data into a peak-based spike encoder of at least one of the spiking neural network to obtain a plurality of encoded spike trains from each spiking neural network based on one or more peaks detected from each ECG time-series data;

providing the plurality of encoded spike trains into at least one of the spiking neural network, wherein a reservoir of the first spiking neural network obtains neuronal trace values of a plurality of recurrently connected excitatory neurons, and wherein a spiking neural network (SNN) layer of the second spiking neural network obtains neuronal trace values of a plurality of neurons connected in a feed forward pattern;

extracting from each ECG time-series data, a first set of spatio-temporal features from the first spiking neural network based on the neuronal trace values of the plurality of excitatory neurons, and a second set of spatio-temporal features from the second spiking neural network based on the neuronal trace values of the plurality of neurons connected in the feed forward pattern;

training the logistic regression-based ECG classifier with one or more class labels associated with each of the spiking neural network by passing corresponding neuronal trace values of the plurality of recurrently connected excitatory neurons into the first neural network and the neuronal trace values of the plurality of neurons connected in the feed forward pattern into the second neural network; and

processing a plurality of test dataset received as input into at least one of the spiking neural network to obtain associated class label from the logistic regression-based ECG classifier.

**14.** The one or more non-transitory machine-readable information storage mediums of claim 13, wherein the peak-based spike encoder of at least one of the spiking neural network obtains the plurality of encoded spike trains by performing the steps of:

filtering the plurality of ECG time-series data by using a low pass filter;

passing the plurality of ECG time-series data through an integrate and fire (IF) neuron model having an initial membrane threshold value;

detecting one or more spike trains of the plurality of ECG time-series data which occurred above a first threshold timestep and discarding one or more spike trains that precede below the first threshold timestep;

identifying if the one or more spike trains of each ECG time-series data comprise a positive slope and a negative slope;

classifying the one or more spike trains of each ECG time-series data as a first wave, if the occurrence of each spike train is within a second threshold timestep and if maximum derivative of each ECG time-series data is lesser than half the maximum derivative of each spike train occurred at the time of previous instance;

classifying the one or more spike trains of each ECG time-series data as a second wave, if the occurrence of each spike train is above the second threshold timestep and if maximum derivative of each ECG time-series data is greater than half the maximum derivative of each spike train occurred at the time of previous instance and if one or more spiking activities of each spike train exceeds a third threshold and otherwise classified as noise; and

adjusting the initial membrane threshold value of the IF neuron model based on the change occurred in the plurality of ECG time-series data and previous spiking activity.

**15.** The one or more non-transitory machine-readable information storage mediums of claim 17, wherein the first spiking neural network comprise a reservoir-based spiking neural network and the second spiking neural network comprise a feed forward based spiking neural network,

wherein the first spiking neural network comprises the peak-based spike encoder, a reservoir, and the logistic regression based ECG classifier, and wherein one or more connections between the peak-based spike encoder and the reservoir is sparse, and

wherein the second spiking neural network comprise the peak-based spike encoder, the feed forward based spiking neural network (SNN) layer, and the logistic regression based ECG classifier, and wherein one or more connections between the peak-based spike encoder and the spiking neural network is fully connected.

System **100**

Hardware Processor(s) **104**

I/O Interface(s) **106**

Memory **102**

Modules **108**

FIG. 1

200

FIG. 2

300

Full connectivity

Input : ECG Time Series

| F(1) | F(2) | F(t)... | F(n)... |

F(t)

Peak-based spike Encoder
302

SNN Layer 304

Logistic regression-
based ECG classifier
306

Output : Class label

FIG. 3

400

obtain by a spiking neural network a training dataset comprising a plurality of ECG time-series data from a plurality of sensors, wherein the spiking neural network comprise at least one of a first neural network and a second neural network

402

train a logistic regression-based ECG classifier associated with at least one of the first neural network and the second neural network with the plurality of ECG time-series data

404

processing a plurality of test dataset received as input into at least one of the spiking neural network to obtain associated class label from the logistic regression-based ECG classifier

406

FIG. 4

FIG. 5

FIG.6

FIG.7

FIG.8

**EP 4 386 622 A1**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DEY SOUNAK ET AL: "Efficient Time Series Classification using Spiking Reservoir", 2022 INTERNATIONAL JOINT CONFERENCE ON NEURAL NETWORKS (IJCNN), IEEE, 18 July 2022 (2022-07-18), pages 1-8, XP034199568, DOI: 10.1109/IJCNN55064.2022.9892728 [retrieved on 2022-09-30] * abstract * * page 1, left-hand column, line 1 – page 7, left-hand column, line 10 * | 1-15 | INV. G06N3/044 G06N3/047 G06N3/049 G06N3/088 |
| A | Anonymous: "Electrocardiography – Wikipedia", , 15 November 2022 (2022-11-15), pages 1-30, XP093160188, Retrieved from the Internet: URL:https://en.wikipedia.org/w/index.php?title=Electrocardiography&oldid=1122094984 [retrieved on 2024-05-07] * page 1, line 1 – page 21, line 3 * | 1-15 | |
| A | BANERJEE DIGHANCHAL DIGHANCHAL B@TCS COM ET AL: "Efficient Optimized Spike Encoding of Multivariate Time-series", 2022 7TH INTERNATIONAL CONFERENCE ON INTELLIGENT INFORMATION TECHNOLOGY, ACMPUB27, NEW YORK, NY, USA, 28 March 2022 (2022-03-28), pages 22-28, XP058853906, DOI: 10.1145/3517343.3517349 ISBN: 978-1-4503-9658-5 * abstract * * page 22, right-hand column, line 1 – page 27, left-hand column, last line * | 1-15 | TECHNICAL FIELDS SEARCHED (IPC) G06N |

-/--

The present search report has been drawn up for all claims

| Place of search | Date of completion of the search | Examiner |
|---|---|---|
| The Hague | 10 May 2024 | Totir, Felix |

page 1 of 2

Europäisches
Patentamt

European
Patent Office

Office européen
des brevets

**EUROPEAN SEARCH REPORT**

Application Number

EP 23 19 7705

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | Dighanchal Banerjee: "An SNN Based ECG Classifier For Wearable Edge Devices", , 2 December 2022 (2022-12-02), pages 1-7, XP093159718, Retrieved from the Internet: URL:https://openreview.net/pdf?id=V8yemRAs00- [retrieved on 2024-05-07] * abstract * * page 1, line 1 - page 5, last line * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 10 May 2024 | Totir, Felix |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 386 622 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221068002